# EUROPEAN PATENT APPLICATION

(11) **EP 4 819 148 A1**
(43) Date of publication of application: **07.10.2026**
(21) Application number: 26161570.2
(22) Date of filing: 02.03.2026
(51) Int. Cl.: G16B 40/20, G16B 15/20, G06N 3/045

(54) **METHOD AND SYSTEM FOR PREDICTING CHANGE IN PROTEIN STABILITY USING NEURAL NETWORK MODEL**

(30) Priority: 30.03.2025 IN 202521031406
(71) Applicant: Tata Consultancy Services Limited, Mumbai, Maharashtra 400 021 (IN)
(72) Inventor: RANA, Sadhna, 751024 Bhubaneswar, Odisha (IN); PRADHAN, Swatantra, 751024 Bhubaneswar, Odisha (IN); SRINIVASAN, Rajgopal, 500032 Hyderabad, Telangana (IN)
(74) Representative: Goddar, Heinz J.

(57) **Abstract**

This disclosure relates generally to a sequence based neural network method for predicting change in stability of proteins upon single point mutations (SPM). The Stateof-the-art methods are unable to capture change in residue environment using protein sequences information alone. The method of the present disclosure considers novel representation of a wild-type protein sequence and a mutant-type protein sequence by considering residue environment in addition to the residue at the position of mutation. Highly attended residues with respect to the position of mutation form residue environment and are identified by leveraging attention mechanism of the Protein Language Model (PLM) used in this study. Thus, the method captures change in the residue neighbour on mutation without using structural information. The method leads to a more accurate prediction of change in free energy (ΔΔG) at the site of mutation. as compared to existing state of the art methods.

## Description

### CROSS-REFERENCE TO RELATED APPLICATIONS AND PRIORITY

The present application claims priority from Indian patent application no. 202521031406, filed on March 30, 2025.

### TECHNICAL FIELD

The disclosure herein generally relates to protein stability prediction, and, more particularly, to a method and system for protein stability prediction using neural network model.

### BACKGROUND

Reliable prediction of protein stability changes caused by point variations is important in protein design and engineering. Protein stability changes have been shown to constitute one of the major underlying molecular mechanisms in several mutation-induced diseases. Understanding how specific mutations in a patient affect protein stability or interactions can identify possible drug resistance/sensitivity in that patient, allowing better therapeutic approaches. In addition, such knowledge is important for improving the protein design through site-directed or random mutagenesis, leading to promising new approaches for precision medicine. Such tools could help clinicians in selecting the most appropriate treatments and creating novel therapeutic strategies based on a more thorough genetic understanding of the patient's disease. Characterizing the molecular consequences of mutations can provide key insights into their biological outcome.

Over the past decades, dozens of structure-based and sequence-based methods have been proposed to understand effect of the mutation in overall stability of the proteins. However, these methods capture only change in residues at the site of mutation and lack features related to changes in space surrounding the mutation site, because they are difficult to obtain. DDMut and I-Mutant are sequence-based methods for protein stability prediction. The DDMut predicts changes in Gibbs Free Energy (ΔΔG) upon single and multiple point mutations with deep learning models built by integrating graph-based representations of the localized 3D environment, with convolutional layers and transformer encoders. This combination better captured the distance patterns between atoms by extracting both short-range and long-range interactions. The I-Mutant is a support vector machine (SVM) based prediction of protein stability changes upon single-site mutations. The tool evaluates the stability change upon single site mutation starting from the protein structure or from the protein sequence.

Both DDMut and I-Mutant incorporate residue environment while predicting the protein stability but do not incorporate change in residue environment due to mutations. Such features are difficult to get as these require modeling of mutant proteins and getting change in various features. Other algorithms such as ThermoNet also capture change in residue environment by using a cumbersome process of modeling structure of the mutant sequences. However, the accuracy of a modeled mutant structure is also limited by the algorithm used for prediction.

### SUMMARY

Embodiments of the present disclosure present technological improvements as solutions to one or more of the above-mentioned technical problems recognized by the inventors in conventional systems. For example, in one embodiment, a method for protein stability prediction using neural network model is provided. The method includes receiving, via one or more hardware processors, (a) a wild-type protein sequence, (b) a mutant-type protein sequence, and (c) a single mutation position (SMP) in the wild-type protein sequence. The method further includes extracting, via the one or more hardware processors, a plurality of features, by processing (a) the wild-type protein sequence, (b) the mutant-type protein sequence, and (c) the SMP in the wild-type protein sequence using a pre-trained protein language model (PLM). The PLM is trained on one or more unlabeled protein sequences from a Protein database in a self-supervised manner using a mask language modeling principle which takes protein sequence as input and gives an appropriate contextual representation for each position in the protein sequence. The method further includes separately generating, via the one or more hardware processors, (i) a first representation matrix and (ii) a first attention matrix, from a plurality of extracted features of the wild-type protein sequence, and (i) a second representation matrix and (ii) a second attention matrix, from the plurality of extracted features of the mutant-type protein sequence. The first representation matrix is a feature vector representation of each amino acid in the wild-type protein sequence, and the second representation matrix is the feature vector representation of each amino acid in the mutant-type protein sequence. The first attention matrix captures an attention paid by each amino acid residue to one or more other amino acids in the wild-type protein sequence, and the second attention matrix captures the attention paid by each amino acid residue to one or more amino acids in the mutant-type protein sequence. The method further includes selecting, using a selection algorithm, via the one or more hardware processors, one or more attention scores, by processing the first representation matrix, the first attention matrix, the second representation matrix, the second attention matrix, and the SMP, wherein the one or more attention scores are selected to obtain a) a plurality of highly attended amino acids from the wild-type protein sequence with respect to a wild-type residue at the SMP, and b) a plurality of highly attended amino acids from the mutant-type protein sequence with respect to a mutant-type residue at the SMP. The attention score represents one or more numerical values assigned to each amino acid of the wild-type protein sequence and the mutant-type protein sequence, and wherein the attention score is utilized in assessing importance of each amino acid of the wild-type protein sequence and the mutant-type protein sequence with respect to a protein stability. The method further includes obtaining, via the one or more hardware processors, a residue environment by computing a first final feature representation, and a second final feature representation. The first final feature representation aggregates one or more vector representations of each of the plurality of highly attended amino acids and a SMP representation of the wild-type protein sequence extracted from the first representation matrix. The second final feature representation aggregates one or more vector representations of each of the plurality of highly attended amino acids and the SMP representation of the mutant-type protein sequence extracted from the second representation matrix. The method further includes computing, via the one or more hardware processors, a difference vector between the first final feature representation, and the second final feature representation, wherein the difference vector represents a change in amino acid residue and a change in residue environment after a single point mutation of the wild-type protein sequence. The method further includes estimating, via the one or more hardware processors, a change in free energy (ΔΔG) by providing the difference vector to a neural-network based prediction block, wherein value of ΔΔG below 0 indicates that the single point mutation is thermodynamically more stable with respect to the wild-type protein sequence.

In another aspect, a system for protein stability prediction using neural network model is provided. The system includes at least one memory storing programmed instructions, one or more Input/Output (I/O) interfaces, and one or more hardware processors, and a neural network model 110 operatively coupled to a corresponding at least one memory. The system is configured to receive (a) a wild-type protein sequence, (b) a mutant-type protein sequence, and (c) a single mutation position (SMP) in the wild-type protein sequence. The system is configured to extract a plurality of features, by processing (a) the wild-type protein sequence, (b) the mutant-type protein sequence, and (c) the SMP in the wild-type protein sequence using a pre-trained protein language model (PLM). The PLM is trained on one or more unlabeled protein sequences from a Protein database in a self-supervised manner using a mask language modeling principle which takes protein sequence as input and gives an appropriate contextual representation for each position in the protein sequence. The system is configured to separately generating, via the one or more hardware processors, (i) a first representation matrix and (ii) a first attention matrix, from a plurality of extracted features of the wild-type protein sequence, and (i) a second representation matrix and (ii) a second attention matrix, from the plurality of extracted features of the mutant-type protein sequence. The first representation matrix is a feature vector representation of each amino acid in the wild-type protein sequence, and the second representation matrix is the feature vector representation of each amino acid in the mutant-type protein sequence. The first attention matrix captures an attention paid by each amino acid residue to one or more other amino acids in the wild-type protein sequence, and the second attention matrix captures the attention paid by each amino acid residue to one or more amino acids in the mutant-type protein sequence. The system is configured to select using a selection algorithm, one or more attention scores, by processing the first representation matrix, the first attention matrix, the second representation matrix, the second attention matrix, and the SMP, wherein the one or more attention scores are selected to obtain a) a plurality of highly attended amino acids from the wild-type protein sequence with respect to a wild-type residue at the SMP, and b) a plurality of highly attended amino acids from the mutant-type protein sequence with respect to a mutant-type residue at the SMP. The attention score represents one or more numerical values assigned to each amino acid of the wild-type protein sequence and the mutant-type protein sequence, and wherein the attention score is utilized in assessing importance of each amino acid of the wild-type protein sequence and the mutant-type protein sequence with respect to a protein stability. The system is configured to obtain a residue environment by computing a first final feature representation, and a second final feature representation. The first final feature representation aggregates one or more vector representations of each of the plurality of highly attended amino acids and a SMP representation of the wild-type protein sequence extracted from the first representation matrix. The second final feature representation aggregates one or more vector representations of each of the plurality of highly attended amino acids and the SMP representation of the mutant-type protein sequence extracted from the second representation matrix. The system is configured to compute a difference vector between the first final feature representation, and the second final feature representation, wherein the difference vector represents a change in amino acid residue and a change in residue environment after a single point mutation of the wild-type protein sequence. The system is configured to estimate a change in free energy (ΔΔG) by providing the difference vector to a neural-network based prediction block, wherein value of ΔΔG below 0 indicates that the single point mutation is thermodynamically more stable with respect to the wild-type protein sequence.

In yet another aspect, there are provided one or more non-transitory machine-readable information storage mediums comprising one or more instructions for protein stability prediction using neural network model is provided. The one or more non-transitory machine-readable information storage mediums when executed by the one or more hardware processors cause: receiving (a) a wild-type protein sequence, (b) a mutant-type protein sequence, and (c) a single mutation position (SMP) in the wild-type protein sequence. The one or more non-transitory machine-readable information storage mediums when executed by the one or more hardware processors cause: extracting a plurality of features, by processing (a) the wild-type protein sequence, (b) the mutant-type protein sequence, and (c) the SMP in the wild-type protein sequence using a pre-trained protein language model (PLM). The PLM is trained on one or more unlabeled protein sequences from a Protein database in a self-supervised manner using a mask language modeling principle which takes protein sequence as input and gives an appropriate contextual representation for each position in the protein sequence. The one or more non-transitory machine-readable information storage mediums when executed by the one or more hardware processors cause: separately generating (i) a first representation matrix and (ii) a first attention matrix, from a plurality of extracted features of the wild-type protein sequence, and (i) a second representation matrix and (ii) a second attention matrix, from the plurality of extracted features of the mutant-type protein sequence. The first representation matrix is a feature vector representation of each amino acid in the wild-type protein sequence, and the second representation matrix is the feature vector representation of each amino acid in the mutant-type protein sequence. The first attention matrix captures an attention paid by each amino acid residue to one or more other amino acids in the wild-type protein sequence, and the second attention matrix captures the attention paid by each amino acid residue to one or more amino acids in the mutant-type protein sequence. The one or more non-transitory machine-readable information storage mediums when executed by the one or more hardware processors cause: selecting using a selection algorithm, one or more attention scores, by processing the first representation matrix, the first attention matrix, the second representation matrix, the second attention matrix, and the SMP, wherein the one or more attention scores are selected to obtain a) a plurality of highly attended amino acids from the wild-type protein sequence with respect to a wild-type residue at the SMP, and b) a plurality of highly attended amino acids from the mutant-type protein sequence with respect to a mutant-type residue at the SMP. The attention score represents one or more numerical values assigned to each amino acid of the wild-type protein sequence and the mutant-type protein sequence, and wherein the attention score is utilized in assessing importance of each amino acid of the wild-type protein sequence and the mutant-type protein sequence with respect to a protein stability. The one or more non-transitory machine-readable information storage mediums when executed by the one or more hardware processors cause: obtaining a residue environment by computing a first final feature representation, and a second final feature representation. The first final feature representation aggregates one or more vector representations of each of the plurality of highly attended amino acids and a SMP representation of the wild-type protein sequence extracted from the first representation matrix. The second final feature representation aggregates one or more vector representations of each of the plurality of highly attended amino acids and the SMP representation of the mutant-type protein sequence extracted from the second representation matrix. The one or more non-transitory machine-readable information storage mediums when executed by the one or more hardware processors cause: computing a difference vector between the first final feature representation, and the second final feature representation, wherein the difference vector represents a change in amino acid residue and a change in residue environment after a single point mutation of the wild-type protein sequence. The one or more non-transitory machine-readable information storage mediums when executed by the one or more hardware processors cause: estimating a change in free energy (ΔΔG) by providing the difference vector to a neural-network based prediction block, wherein value of ΔΔG below 0 indicates that the single point mutation is thermodynamically more stable with respect to the wild-type protein sequence.

It is to be understood that both the foregoing general description and the following detailed description are exemplary and explanatory only and are not restrictive of the invention, as claimed.

### BRIEF DESCRIPTION OF DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of a system 100 for protein stability prediction using neural network model, according to some embodiments of the present disclosure.
FIG. 2 illustrates an overall framework for protein stability prediction through neural network model based estimation of change in free energy, using the system of FIG. 1, according to some embodiments of the present disclosure.
FIGS. 3A, 3B and 3C are flow diagrams of an illustrative method 300 for neural network model prediction of protein stability, using the system of FIG. 1, according to some embodiments of the present disclosure.

### DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present disclosure relates to a sequence based method for predicting protein stability. The method involves leveraging an attention mechanism of ESM-2 to define residue neighbors (amino acid residues from the protein sequence) of the position of interest (i.e. single mutation position) and use only top attended neighboring residues for representing the wild-type protein sequence and mutant-type protein sequence as opposed to average representation or single residue representation of the protein at the point of mutation. Considering the approaches for proteins, ESM family architectures, developed by the MetaAI group, with the most recent version being ESM-2, are among the state-of-the-art approaches in various tasks, such as protein function prediction, protein family annotations, and protein sequence conservation. The ESM-2 architectures are pre-trained on billions of protein sequences.

This disclosure relates generally to protein stability prediction in proteins with single mutation using neural network (NN) modelswith the method illustrates a novel way to the represent wild-type protein sequence and the mutant-type protein in the neural network model by considering the "functional" residue neighbours around the mutation site leveraging attention mechanism of ESM-2, a transformer model trained on protein sequence data. In the ESM-2, the attention mechanism operates by essentially "paying attention" to the most relevant interactions between residues, which is key to its ability to learn complex patterns in protein sequences. Thus, a residue environment is obtained by getting residues that have high attention scores to the mutation site. Thus, instead of using difference in amino acids at the site of mutation to train the neural network model as is usually done by majority of the methods, the present method utilizes group of residues to represent the site of mutation. This group contains the residue that is mutated as well as residues that are highly attended by the mutated residue.

The method involves a unique way of representing protein sequences that offers the following advantages:
1. The neural network model takes input the SMP representation and the SMP's important neighbors representations for calculating ΔΔG. This meticulous selection process helps the model to learn better mapping as compared to the previous state of the art methods where all the residues representation were considered.
2. The SMP residue environment derived from top attention scores has the information of local neighborhood and the global neighborhood in the protein's sequence as well as structural space.

As used herein, the term *'single mutation position'* refers to a specific location in a mutant-type protein sequence where a single amino acid has been changed by substitution in the wild-type protein sequence.

As used herein, the term *'wild-type protein sequence'* refers to the most prevalent and functionally normal form of a protein that serves as a baseline for comparison against a mutant or altered form.

As used herein, the term *'mutant-type protein sequence'* refers to an altered amino acid sequence of a wildtype protein sequence. As used herein, the term *'first representation matrix'* refers to a feature vector representation of each amino acid in the wild-type protein sequence.

As used herein, the term *'second representation matrix'* refers to a feature vector representation of each amino acid in the mutant-type protein sequence.

As used herein, the term *'first attention matrix'* refers to a representation that captures an attention paid by each amino acid residue to one or more other amino acids in the wild-type protein sequence.

As used herein, the term *'second attention matrix'* refers to a representation that captures an attention paid by each amino acid residue to one or more other amino acids in the mutant-type protein sequence.

As used herein, the term *'attention score'* refers to one or more numerical values assigned to each amino acid of the wild-type protein sequence and the mutant-type protein sequence, and wherein the attention score is utilized in assessing importance of each amino acid of the wild-type protein sequence and the mutant-type protein sequence for inferring residue neighbors.

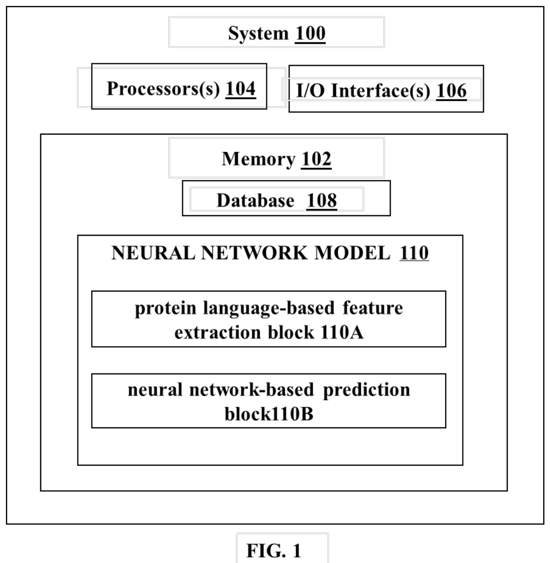

Referring now to the drawings, and more particularly to FIG. 1 through FIG. 3C, where similar reference characters denote corresponding features consistently throughout the figures, there are shown preferred embodiments, and these embodiments are described in the context of the following exemplary system and/or method.

FIG. 1 illustrates an exemplary block diagram of a system 100 for protein stability prediction using neural network model, according to some embodiments of the present disclosure.

In an embodiment, the system 100 includes a processor(s) 104,communication interface device(s) 106, alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100. Referring to the components of system 100, in an embodiment, the processor(s) 104, can be one or more hardware processors 104. In an embodiment, the one or more hardware processors 104 can be implemented as one or more microprocessors microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102.

In an embodiment, the system 100 can be implemented in a variety of computing systems including laptop computers, notebooks, hand-held devices such as mobile phones, workstations, mainframe computers, servers, and the like. The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like.

In an embodiment, the I/O interface (s) 106 can include one or more ports for connecting to number of external devices or to another server or devices. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

In an embodiment, the memory 102 includes a neural network model 110. The neural network model 110 comprises a protein language-based feature extraction block 110A and a neural network-based prediction block 110B.

The protein language-based feature extraction block 110A receives a wild-type protein sequence, a mutant-type protein sequence, and a single mutation position (SMP) in the wild-type protein sequence. A pre-trained protein language model (a ESM-2 model) takes up a wild-type protein sequence, a mutant-type protein sequence separately and generates their respective representations along with auxiliary outputs such as attention matrix for each of them. The ESM-2 model extracts a plurality of features, by processing the wild-type protein sequence, the mutant-type protein sequence, and the SMP in the wild-type protein sequence using a pre-trained protein language model. The plurality of features extracted from the wild-type protein sequence are utilized to generate a first representation matrix and a first attention matrix. Similarly, the plurality of features extracted from the mutant-type protein sequence are utilized to generate a second representation matrix and a second attention matrix. The protein language-based feature extraction block 110A predicts a residue environment by identifying one or more highly attended amino acid residues from the wild-type protein sequence and the mutant-type protein sequence. The protein language-based feature extraction block 110A utilizes a selection algorithm to select one or more attention scores, by processing the first representation matrix, the first attention matrix, the second representation matrix, the second attention matrix, and the SMP. The one or more attention scores are selected to obtain a) a plurality of highly attended amino acids from the wild-type protein sequence with respect to a wild-type residue at the SMP, and b) a plurality of highly attended amino acids from the mutant-type protein sequence with respect to a mutant-type residue at the SMP. The residue environment is obtained environment by computing a first final feature representation, and a second final feature representation. The neural network-based prediction block 110B computes a difference vector between the first final feature representation, and the second final feature representation. The difference vector represents a change in amino acid residue and a change in residue environment after a single point mutation of the wild-type protein sequence. The difference vector is provided to the neural-network based prediction block 110B. The neural-network based prediction block 110B estimates a change in free energy (ΔΔG) based on the difference vector. Value of ΔΔG below 0 indicates that the single point mutation is stable with respect to the wild-type protein sequence.

The memory 102 further comprises of a plurality of modules that includes programs or coded instructions that supplement applications or functions performed by the system 100 for executing different steps involved in the micro-batch processing, being performed by the system 100. The modules, amongst other things, can include routines, programs, objects, components, and data structures, which perform particular tasks or implement particular abstract data types. The modules may also be used as signal processor(s), node machine(s), logic circuitries, and/or any other device or component that manipulates signals based on operational instructions. Further, the modules can be used by hardware, by computer-readable instructions executed by the one or more hardware processors 104, or by a combination thereof. The modules may include computer-readable instructions that supplement applications or functions performed by the system 100. Further, the memory 102 may comprise information pertaining to input(s)/output(s) of each step performed by the processor(s) 104 of the system100 and methods of the present disclosure. Further, the memory 102 includes a database 108. The database (or repository) 108 may include a plurality of abstracted piece of code for refinement and data that is processed, received, or generated as a result of the execution of the plurality of modules. The external database is communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

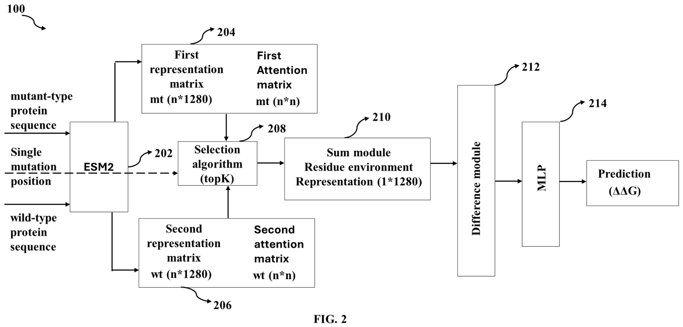

FIG. 2 illustrates an overall framework for protein stability prediction through neural network model based estimation of change in free energy, using the system of FIG. 1, according to some embodiments of the present disclosure.

As illustrated in the FIG. 2, protein stability prediction due to single point mutation involves a protein language model (PLM), ESM-2. At 202, the ESM-2 receives the mutant-type protein sequence, the wild-type protein sequence, and the SMP in the wild-type protein sequence. The ESM-2 processes the mutant-type protein sequence, the wild-type protein sequence and the SMP in the wild-type protein sequence to extract a plurality of features. At 204, from a plurality of extracted features of the wild-type protein sequence a first representation matrix and a first attention matrix is generated. The first representation matrix is of size N* 1280 where N is the length of the amino acid sequence of the wild-type protein and 1280 is the feature vector representing each amino acid in the wild-type protein sequence. The attention matrix is of size N*N , which shows how much each amino acid residue in a wild-type protein sequence pays attention to other amino acids. Similarly, at 206, from a plurality of extracted features of the mutant-type protein sequence a second representation matrix and a second attention matrix is generated. The second representation matrix is of size N* 1280 where N is the length of the amino acid sequence of the mutant-type protein and 1280 is the feature vector representing each amino acid in the mutant-type protein sequence. The attention matrix is of size N*N , which shows how much each amino acid residue in a mutant-type protein sequence pays attention to other amino acids.

At 208, the selection algorithm processes the first representation matrix and the first attention matrix using the selection algorithm to select one or more attention scores. The one or more attention scores are selected for each amino acid in the wild-type protein sequence with respect to the amino acid representing the single mutation position. Based on the one or more attention score, a plurality of highly attended amino acids from the wild-type protein sequence with respect to a mutant-type residue at the SMP are screened. Similarly, at 208, the selection algorithm processes the second representation matrix and the second attention matrix using the selection algorithm to select one or more attention scores. The one or more attention scores are selected for each amino acid in the mutant-type protein sequence with respect to the amino acid representing the single mutation position. Based on the one or more attention score, a plurality of highly attended amino acids from the mutant-type protein sequence with respect to a mutant-type residue at the SMP are screened.

At 210, a residue environment is created by computing a first final feature representation, and a second final feature representation. The first final feature representation aggregates one or more vector representations of each of the plurality of highly attended amino acids and a SMP representation of the wild-type protein sequence extracted from the first representation matrix. Similarly, the second final feature representation aggregates one or more vector representations of each of the plurality of highly attended amino acids and the SMP representation of the mutant-type protein sequence extracted from the second representation matrix. At 212, a difference module computes a difference vector between the first final feature representation, and the second final feature representation. The difference vector represents a change in amino acid residue and a change in residue environment after a single point mutation of the wild-type protein sequence. Therefore a final representation is the difference between the first final feature representation of the wild-type protein sequence,, and the second final feature representation of the mutant-type protein sequence.

At 214, a change in free energy (ΔΔG) is estimated by providing the difference vector to a neural-network based prediction block. The difference vector which is the final representation is passed as a feature to a multi-layer perceptron (MLP) network (the prediction block)to output the change in free energy (ΔΔG). Value of ΔΔG below 0 indicates that the single point mutation is stable with respect to the wild-type protein sequence.

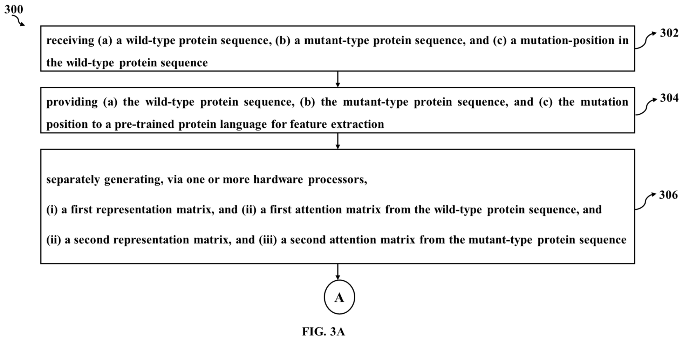

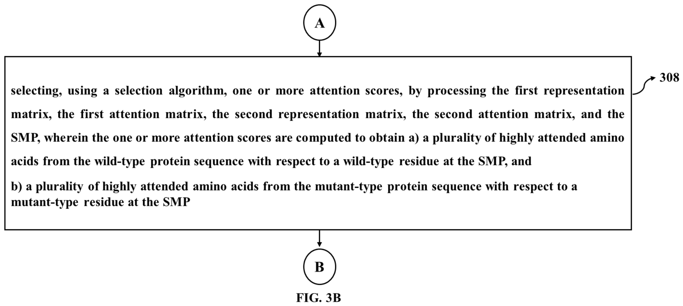

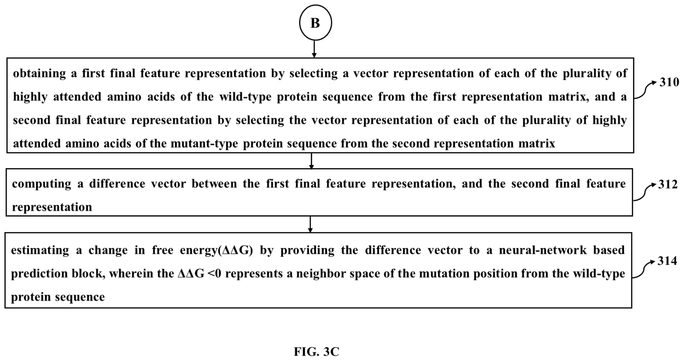

FIGS. 3A, 3B and 3C are flow diagrams of an illustrative method 300 for neural network model prediction of protein stability, using the system of FIG. 1, according to some embodiments of the present disclosure.

The steps of the method 300 of the present disclosure will now be explained with reference to the components or blocks of the system 100 as depicted in FIG. 1. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practically. Further, some steps may be performed simultaneously.

At step 302 of the method 300, the one or more hardware processors 104 are configured to receive (a) a wild-type protein sequence, (b) a mutant-type protein sequence, and (c) a single mutation position (SMP) in the wild-type protein sequence. the most prevalent and functionally normal form of a protein that serves as a baseline for comparison against a mutant or altered form. The mutant-type protein sequence has an altered amino acid sequence of a wildtype protein sequence.

At step 304 of the method 300, the one or more hardware processors 104 are configured to extract a plurality of features, by processing (a) the wild-type protein sequence, (b) the mutant-type protein sequence, and (c) the SMP in the wild-type protein sequence using a pre-trained protein language model (PLM). The pre-trained protein language model is an ESM-2 model with a stability prediction module. The ESM-2 model first extracts the plurality of features and then processes the plurality of extracted features. The PLM trained on one or more unlabeled protein sequences from a Protein database in a self-supervised manner using a mask language modeling principle which takes protein sequence as input and gives an appropriate contextual representation for each position in the protein sequence. The UNIREF (UniProt Reference Clusters (UniRef) provide clustered sets of sequences from the UniProt Knowledgebase (including isoforms) and selected UniParc records in order to obtain complete coverage of the sequence space at several resolutions while hiding redundant sequences.

At step 306 of the method 300, the one or more hardware processors 104 are configured to separately generate (i) a first representation matrix and (ii) a first attention matrix, from a plurality of extracted features of the wild-type protein sequence, and (i) a second representation matrix and (ii) a second attention matrix, from the plurality of extracted features of the mutant-type protein sequence. The wild-type protein sequence and the mutant-type protein sequence are passed to the ESM-2 model as the plurality of extracted features to get one or more representations for the wild-type protein sequence and the mutant-type protein sequence. The first representation matrix is derived from the extracted features of the wild-type protein sequence. The first representation matrix is a feature vector representation of each amino acid in the wild-type protein sequence. Similarly, the second representation matrix is derived from the extracted features of the mutant-type protein sequence. The second representation matrix is a feature vector representation of each amino acid in the mutant-type protein sequence. The first attention matrix captures an attention paid by each amino acid residue to one or more other amino acids in the wild-type protein sequence, and the second attention matrix captures the attention paid by each amino acid residue to one or more amino acids in the mutant-type protein sequence.

At step 308 of the method 300, the one or more hardware processors 104 are configured to select , using a selection algorithm, one or more attention scores, by processing the first representation matrix, the first attention matrix, the second representation matrix, the second attention matrix, and the SMP. The one or more attention scores are selected to obtain a) a plurality of highly attended amino acids from the wild-type protein sequence with respect to a wild-type residue at the SMP, and b) a plurality of highly attended amino acids from the mutant-type protein sequence with respect to a mutant-type residue at the SMP. The attention score represents one or more numerical values assigned to each amino acid of the wild-type protein sequence and the mutant-type protein sequence. The attention score is utilized in assessing importance of each amino acid of the wild-type protein sequence and the mutant-type protein sequence with respect to a protein stability.

At step 310 of the method 300, the one or more hardware processors 104 are configured to obtain a residue environment by computing a first final feature representation, and a second final feature representation. The first final feature representation aggregates one or more vector representations of each of the plurality of highly attended amino acids and a SMP representation of the wild-type protein sequence extracted from the first representation matrix. The second final feature representation aggregates one or more vector representations of each of the plurality of highly attended amino acids and the SMP representation of the mutant-type protein sequence extracted from the second representation matrix. From the amino acid representations of the wild-type protein sequence and the mutant-type protein sequence, only the topK amino acid representations are considered to obtain the final representation. TopK amino acids representations are obtained based on the attention scores (i.e., for the mutation residue position, topK attended residues from the attention matrix are obtained). These topK amino acids representations are summed up to get final representations for wild-type and mutant-type amino acid protein sequences respectively.

At step 312 of the method 300, the one or more hardware processors 104 are configured to compute a difference vector between the first final feature representation, and the second final feature representation. The difference vector represents a change in amino acid residue and a change in residue environment after a single point mutation of the wild-type protein sequence. The vector difference of mutant-type and wild-type amino acid sequences indicates a final representation. The final feature representation includes the change in amino acid residue at the single mutation position as well as the change in residue environment indicated by the plurality of highly attended amino acids. This inclusive approach results into an improvement of the reliability of the PLM-based prediction. Modelling of the residue environment through representation matrix and attention matrix enhances model interpretability.

At step 314 of the method 300, the one or more hardware processors 104 are configured to estimate a change in free energy (ΔΔG) by providing the difference vector to a neural-network based prediction block. The value of ΔΔG below 0 indicates that the single point mutation is favorable with respect to the wild-type protein sequence. The value of ΔΔG above 0 indicates that the single point mutation is not favorable with respect to the wild-type protein sequence. This results in better protein stability prediction because estimation of the change in free energy is inclusive of change in amino acid residue at the site of mutation as well as the residue environment represented by the highly attended amino acids.

### MODEL TRAINING AND PERFORMANCE TESTING

The Neural Network (NN) model 110 is trained on a publicly available dataset. To train the model, the publicly available dataset is undergone pre-processing for data curation by the following operations:
**1.** Removed the records (wild-type protein sequence, mutant-type protein sequence and the corresponding single mutation position) having tag "unreliable".
2. Removed the records when no mutation is introduced.
3. Removed the records associated with insertions and/or deletions.
4. Removed the records associated with multiple mutations.

The sequences of the wild-type proteins from the mutant-type protein sequence are reconstructed in the "aa_seq" column and the mutation in the column "mut_type" of the original dataset. The effect of mutation from the column "ddG_ML" is multiplied by -1 to convert the values into folding free energy changes (negative values denote stabilization). The final dataset is divided into training, validation and test sets using a 80:10:10 split. The split is done is such a manner that none of the protein sequences which are in train and validation are more than 35% similar to each other and the protein sequences from train set are no more than 25% similar to the sequences in test set. Additionally, protein sequences from the training data which are more than 25% similar to the independent test set S669 are removed. The S669 test set is the benchmark dataset which is used to evaluate the performance of the NN model 110 against the existing algorithms. The NN model 110 consists of a pre-trained PLM ESM-2and a stability prediction module. The wild-type amino acid sequences and mutant-type amino acid sequences of the protein are passed as a feature to the ESM-2 to get the representations for the sequences. From the amino acid representations of the wild-type and mutant-type protein sequences, only the topK amino acid representations are considered to obtain the final representation. TopK amino acids representations are obtained based on the attention scores (i.e., for the mutation residue position, topK attended residues from the attention matrix are obtained). These topK amino acids representations are summed up to get the final representation for wild-type and mutant-type amino acid sequences. The final representation is the difference of the representations of mutant-type and wild-type amino acid sequences. This final representation is passed as a feature to the multi-layer perceptron (MLP) network to output the change in free energy (ΔΔG).

The NN model's 110 trainable parameters are trained for up to 100 epochs using the Adam optimizer with the default learning rate 1e-3 and a training and validation batch size of 8 data points. For the regularization, an early stopping criterion is used with a patience value of 7. The loss monitored during training is L1 loss. The best model after each epoch were saved based on the Pearson correlation metric on the validation set. Next, effect of different training datasets on the NN model 110 performance is analyzed to discriminate between contributions of model architecture and dataset composition. It is observed that the performance of the NN model 110 doesn't increase much when additionally trained on datasets such as S2648, Thermomut and the like. The NN model 110 performance is compared with other state-of-art models. The comparison is presented in Table-1. Pearson's correlations coefficient (PCC) and root mean square error (RMSE) between the experimental and estimated ΔΔGs are computed. The ΔΔGs are predicted by 19 commonly used protein stability prediction tools and NN model 110, on the direct variants of the S669 dataset. The correlations and RMSE are calculated on "Destabilizing" and "Stabilizing" class separately and also on the whole dataset ("Total") and finally only on the destabilizing and stabilizing variants, excluding the neutral ("Non-neutral").

The method of the present achieved a PCC of 0.48 and RMSE of 1.51 which is better that all the ΔΔG predictors considered for comparison. The NN model 110 also achieved a consistent performance across both the classes of stabilizing and destabilizing variants. The NN model 110 also beats all the ΔΔG predictors in terms of the PCC metric on non-neutral variants. The consistency in the performance of the NN model 110 across different classes of variants on the S669 dataset shows that the NN model 110 is more robust and capable of predicting mutation effects on stability changes across a broader group of proteins.

**Table-1**

| Methods/Models | Pearson/RMSE | | | |
|---|---|---|---|---|
| | Total | Destabilizing | Stabilizing | Non-neutral |
| Method of Present disclosure (NN model 110-PTSPred-Arch5 PLM) | 0.48/1.51 | 0.32/1.72 | 0.17/1.97 | 0.46/1.77 |
| ACDC-NN | 0.46/1.49 | 0.34/1.60 | 0.09/2.14 | 0.44/1.71 |
| ThermoMPNN | 0.43/1.52 | -/- | -/- | -/- |
| INPS3D | 0.43/1.50 | 0.35/1.40 | 0.02/2.55 | 0.42/1.67 |
| DDGun3D | 0.43/1.60 | 0.32/1.69 | 0.13/2.22 | 0.41/1.80 |
| INPS-Seq | 0.43/1.52 | 0.26/1.56 | 0.13/2.25 | 0.42/1.70 |
| ACDC-NN-Seq | 0.42/1.53 | 0.28/1.64 | 0.07/2.18 | 0.40/1.75 |
| PremPS | 0.41/1.51 | 0.43/1.48 | -0.08/2.53 | 0.04/1.72 |
| PopMusic | 0.41/1.51 | 0.37/1.40 | 0.09/2.67 | 0.39/1.69 |
| DUET | 0.41/1.52 | 0.34/1.48 | 0.10/2.54 | 0.38/1.72 |
| Dynamut | 0.41/1.60 | 0.32/1.81 | 0.29/2.00 | 0.40/1.85 |
| SDM | 0.41/1.67 | 0.33/1.81 | 0.09/2.14 | 0.40/1.88 |
| DDGun | 0.40/1.75 | 0.25/1.75 | 0.11/2.46 | 0.39/1.90 |
| ABYSSAL | 0.37/- | -/- | -/- | -/- |
| SAAFEC-Seq | 0.36/1.54 | 0.31/1.48 | 0.07/2.60 | 0.34/1.74 |
| mCSM | 0.36/1.54 | 0.30/1.42 | 0.06/2.73 | 0.33/1.73 |
| I-Mutant3.0 | 0.36/1.54 | 0.31/1.48 | 0.07/2.60 | 0.34/1.74 |
| I-Mutant3.0-Seq | 0.34/1.56 | 0.23/1.53 | 0.21/2.53 | 0.33/1.75 |
| MuPro | 0.25/1.61 | 0.19/1.45 | -0.01/2.84 | 2.41/1.78 |
| FoldX | 0.2112.32 | 0.20/2.25 | 0.17/2.66 | 0.24/2.33 |

### USE CASE SCENARIO-I:

An example scenario depicting the method of predicting change in protein stability performed by the disclosed system 100 for predicting protein stability due to single mutation is described below. A protein sequence is taken from a protein data bank with an identification no. as: PDB ID:2O2W chain A having wild-type and mutant-type representations as:

The wild-type protein sequence:

The mutant-type protein sequence:

GIDPFTAEAIAKFNFNGDTQVEMSFRKGERITLLRQVDENWYEGRIPGTSRQG
IFPITYVDVIKRPL

The single mutation position (SMP): G7A

### Protein sequence length: 67

These protein sequences are provided to the PLM for feature extraction.

The extracted features are processed by the PLM to generate a first representation matrix, and a first attention matrix from the wild-type protein sequence as:
First representation matrix: ${\left.R\right|}_{wt}^{67 ∗ 1280}$ wild-type representation matrix is of dimension 67*1280
First attention matrix: ${\left.A\right|}_{wt}^{67 ∗ 67}$ wild-type attention matrix is of dimension 67*67

Similarly, the extracted features are processed by the PLM to generate a second representation matrix, and a second attention matrix from the wild-type protein sequence as:
Second representation matrix: ${\left.R\right|}_{mt}^{67 ∗ 1280}$ mutant-type representation matrix is of dimension 67* 1280
Second attention matrix: ${\left.A\right|}_{mt}^{67 ∗ 67}$ mutant-type attention matrix is of dimension 67*67

By utilizing the selection algorithm, a plurality of highly attended residues from the wild-type protein sequence as well as from the mutant-type protein sequence are screened as:
Highly attended amino acids from wild-type : A9, F5, P4, L33, G44, D3, A11, G7, K64, Y42, I2, I10, Q36, V62, G1
Highly attended amino acids from mutant-type : A9, L33, F5, P4, A7, G44, D3, I2, I10, K64, A11, Q36, Y42, V62, G1

A residue environment is obtained by computing a first final feature representation, and a second final feature representation as: ${{f}_{_}}^{1280} = Σ \left({R}_{wt}\left[\left[9, 5, 4, 33, 44, 3, 11, 7, 64, 42, 2, 10, 36, 62, 1\right],:\right]\right.$ (1) First final feature representation vector : *f¹²⁸⁰* first final feature representation vector has 1280 features ${{s}_{_}}^{1280} = Σ \left({R}_{mt}\left[\left[9, 33, 5, 4, 7, 44, 3, 2, 10, 64, 11, 36, 42, 62, 1\right],:\right]\right.$ (2) Second final feature representation vector: *s*¹²⁸⁰ second final feature representation vector has 1280 features
a difference vector is computed between the first final feature representation, and the second final feature representation as: ${{d}_{_}}^{1280} = {{s}_{_}}^{1280} - {{f}_{_}}^{1280}$ (3)
Difference vector: *d¹²⁸⁰* the difference vector has 1280 features

Finally, a change in free energy (ΔΔG) is computed by providing the difference vector to a neural-network based prediction block, wherein the ΔΔG <0 means that the single point mutation is favorable with respect to the wild-type protein sequence. Final prediction ΔΔG: -0.7919 Kcal/mol implying mutation as thermodynamically more stable. Hence the ΔΔG: -0.7919 Kcal/mol value indicates that the change caused due to mutation at G7A is leading to the thermodynamically more stable protein.

### USE CASE SCENARIO-II:

Another example scenario depicting the method of predicting change in protein stability performed by the disclosed system 100 for predicting protein stability due to single mutation is described below. A protein sequence is taken from a protein data bank with an identification no. as: PDB 1LP1 chain A having wild-type and mutant-type representations as:
The wild-type protein sequence:
The mutant-type protein sequence: 
KFNKELSVAGREIVTLPNLNDPQKKAEIFSLWDDPSQSANLLAEAKKLNDAQ
APK
The single mutation position (SMP): F27E
Protein sequence length: 55
These protein sequences are provided to the PLM for feature extraction.

The extracted features are processed by the PLM to generate a first representation matrix, and a first attention matrix from the wild-type protein sequence as:
First representation matrix: ${\left.R\right|}_{wt}^{55 ∗ 1280}$ wild-type representation matrix is of dimension 55*1280
First attention matrix: ${\left.A\right|}_{wt}^{55 ∗ 55}$ wild-type attention matrix is of dimension 55*55

Similarly, the extracted features are processed by the PLM to generate a second representation matrix, and a second attention matrix from the wild-type protein sequence as:
Second representation matrix: ${\left.R\right|}_{mt}^{55 ∗ 1280}$ mutant-type representation matrix is of dimension 55*1280
Second attention matrix: ${\left.A\right|}_{mt}^{55 ∗ 55}$ mutant-type attention matrix is of dimension 55*55

By utilizing the selection algorithm, a plurality of highly attended residues from the wild-type protein sequence as well as from the mutant-type protein sequence are screened as:
Highly attended amino acids from wild-type : K24, S30, L19, P22, Q23, N20, L16, D21, N18, I13, A45, L31, K25, L48, F27
Highly attended amino acids from mutant-type : K24, S30, Q23, D21, P22, E27, L31, F29, N18, K25, L19, W32, L48, A45, N20

A residue environment is obtained by computing a first final feature representation, and a second final feature representation as: ${{f}_{_}}^{1280} = \sum \left({R}_{wt}\left[\left[24, 30, 19, 22, 23, 20, 16, 21, 18, 13, 45, 31, 25, 48, 27\right],:\right]\right.$ (4) First final feature representation vector : *f¹²⁸⁰* first final feature representation vector has 1280 features ${{s}_{_}}^{1280} = \sum \left({R}_{wt}\left[\left[24, 30, 23, 21, 22, 27, 31, 29, 18, 25, 19, 32, 48, 45, 20\right],:\right]\right.$ (5) Second final feature representation vector: *s*¹²⁸⁰ second final feature representation vector has 1280 features
a difference vector is computed between the first final feature representation, and the second final feature representation as: ${{d}_{_}}^{1280} = {{s}_{_}}^{1280} - {{f}_{_}}^{1280}$ (6) Difference vector: *d¹²⁸⁰* the difference vector has 1280 features

Finally, a change in free energy (ΔΔG) is computed by providing the difference vector to a neural-network based prediction block, wherein the ΔΔG <0 means that the single point mutation is favorable with respect to the wild-type protein sequence. Final prediction ΔΔG: -1.56 Kcal/mol. The final predicted value is close to the experimental ΔΔG=-1.5508 Kcal/mol. Hence the predicted ΔΔG: -1.56 Kcal/mol value indicates that the change caused due to mutation at F27E is leading to the thermodynamically more stable protein.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the present disclosure or if they include equivalent elements within substantial differences from the literal language of the embodiments described herein.

Therefore, the present disclosure employs a method of protein stability prediction in proteins with single mutation using neural network (NN) models. Reliable prediction of protein stability changes caused by point mutations is important in protein design and engineering. Protein stability changes have been shown to constitute one of the major underlying molecular mechanisms in several mutation-induced diseases. Understanding how specific mutations in a patient affect protein stability or interactions can identify possible drug resistance/sensitivity in that patient, allowing better therapeutic approaches. In addition, such knowledge is important for improving the protein design through site-directed or random mutagenesis, leading to promising new approaches for precision medicine. Such tools could help clinicians in selecting the most appropriate treatments and creating novel therapeutic strategies based on a more thorough genetic understanding of the patient's disease. Over the past decades, dozens of structure-based and sequence-based methods have been proposed, showing moderate prediction performance. Most of the methods have only captured changes in residue at the site of mutation and none of the approaches have captured change in the residue environment. Most predictors lack features related to changes in the space surrounding the mutation site, because they are difficult to obtain, given that it is necessary to know the structure of mutant protein to have this information. Since the inclusion of these features could represent an improvement of the reliability of this kind of predictors, the present disclosure involves a method that utilizes a novel representation around the site of mutation. This approach has helped us to better predict the change in free energy at the site of mutation than most of the existing state of the art algorithms. There are two advantages of our approach, first no feature engineering has been used and second, residue environment has been modelled that resulted into better model predictability.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300) for predicting change in protein stability, the method comprising:
receiving (302), via one or more hardware processors, (a) a wild-type protein sequence, (b) a mutant-type protein sequence, and (c) a single mutation position (SMP) in the wild-type protein sequence;
extracting (304), via the one or more hardware processors, a plurality of features, by processing (a) the wild-type protein sequence, (b) the mutant-type protein sequence, and (c) the SMP in the wild-type protein sequence using a pre-trained protein language model;
separately generating (306), via the one or more hardware processors,
(i) a first representation matrix and (ii) a first attention matrix, from a plurality of extracted features of the wild-type protein sequence, and
(i) a second representation matrix and (ii) a second attention matrix, from the plurality of extracted features of the mutant-type protein sequence;
selecting (308), using a selection algorithm, via the one or more hardware processors, one or more attention scores, by processing the first representation matrix, the first attention matrix, the second representation matrix, the second attention matrix, and the SMP, wherein the one or more attention scores are computed to obtain
a) a plurality of highly attended amino acids from the wild-type protein sequence with respect to a wild-type residue at the SMP, and
b) a plurality of highly attended amino acids from the mutant-type protein sequence with respect to a mutant-type residue at the SMP;
obtaining (310), via the one or more hardware processors, a residue environment by computing a first final feature representation, and a second final feature representation wherein,
the first final feature representation aggregates one or more vector representations of each of the plurality of highly attended amino acids and a SMP representation of the wild-type protein sequence extracted from the first representation matrix, and
the second final feature representation aggregates one or more vector representations of each of the plurality of highly attended amino acids and the SMP representation of the mutant-type protein sequence extracted from the second representation matrix;
computing (312), via the one or more hardware processors, a difference vector between the first final feature representation, and the second final feature representation, wherein the difference vector represents a change in amino acid residue and a change in residue environment after a single point mutation of the wild-type protein sequence; and
estimating (314), via the one or more hardware processors, a change in free energy (ΔΔG) by providing the difference vector to a neural-network based prediction block, wherein value of ΔΔG below 0 indicates that the single point mutation is stable with respect to the wild-type protein sequence.

2. The processor implemented method as claimed in claim 1, wherein the first representation matrix is a feature vector representation of each amino acid in the wild-type protein sequence, and the second representation matrix is the feature vector representation of each amino acid in the mutant-type protein sequence.

3. The processor implemented method as claimed in claim 1, wherein the first attention matrix captures attention paid by each amino acid residue to one or more other amino acids in the wild-type protein sequence, and the second attention matrix captures attention paid by each amino acid residue to one or more amino acids in the mutant-type protein sequence.

4. The processor implemented method as claimed in claim 1, wherein the attention score represents one or more numerical values assigned to each amino acid of the wild-type protein sequence and the mutant-type protein sequence, and wherein the attention score is utilized in assessing importance of each amino acid of the wild-type protein sequence and the mutant-type protein sequence with respect to a protein stability.

5. The processor implemented method as claimed in claim 1, wherein the pre-trained model is a protein language model (PLM) trained on one or more unlabeled protein sequences from a Protein database in a self-supervised manner using a mask language modeling principle which takes protein sequence as input and gives an appropriate contextual representation for each position in the protein sequence.

6. The processor implemented method as claimed in claim 1, wherein the wild-type protein sequence, the mutant-type protein sequence, and the SMP in the wild-type protein sequence are processed by a neural network model comprising a protein language based feature extraction block and a neural network based prediction block predict protein stability by measuring the change in free energy (ΔΔG).

7. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive (a) a wild-type protein sequence, (b) a mutant-type protein sequence, and (c) a single mutation position (SMP) in the wild-type protein sequence;
extract a plurality of features, by processing (a) the wild-type protein sequence, (b) the mutant-type protein sequence, and (c) the SMP in the wild-type protein sequence using a pre-trained protein language model;
separately generate
(i) a first representation matrix and (ii) a first attention matrix, from a plurality of extracted features of the wild-type protein sequence, and
(i) a second representation matrix and (ii) a second attention matrix, from the plurality of extracted features of the mutant-type protein sequence;
select, using a selection algorithm, via the one or more hardware processors, one or more attention scores, by processing the first representation matrix, the first attention matrix, the second representation matrix, the second attention matrix, and the SMP, wherein the one or more attention scores are selected to obtain
a) a plurality of highly attended amino acids from the wild-type protein sequence with respect to a wild-type residue at the SMP, and
b) a plurality of highly attended amino acids from the mutant-type protein sequence with respect to a mutant-type residue at the SMP;
obtain, a residue environment by computing a first final feature representation, and a second final feature representation wherein,
the first final feature representation aggregates one or more vector representations of each of the plurality of highly attended amino acids and a SMP representation of the wild-type protein sequence extracted from the first representation matrix, and
the second final feature representation aggregates one or more vector representations of each of the plurality of highly attended amino acids and the SMP representation of the mutant-type protein sequence extracted from the second representation matrix;
compute a difference vector between the first final feature representation, and the second final feature representation, wherein the difference vector represents a change in amino acid residue and a change in residue environment after a single point mutation of the wild-type protein sequence; and
estimate a change in free energy (ΔΔG) by providing the difference vector to a neural-network based prediction block, wherein value of ΔΔG below 0 indicates that the single point mutation is stable with respect to the wild-type protein sequence.

8. The system as claimed in claim 7, wherein the first representation matrix is a feature vector representation of each amino acid in the wild-type protein sequence, and the second representation matrix is the feature vector representation of each amino acid in the mutant-type protein sequence.

9. The system as claimed in claim 7, wherein the first attention matrix captures an attention paid by each amino acid residue to one or more other amino acids in the wild-type protein sequence, and the second attention matrix captures the attention paid by each amino acid residue to one or more amino acids in the mutant-type protein sequence.

10. The system as claimed in claim 7, wherein the attention score represents one or more numerical values assigned to each amino acid of the wild-type protein sequence and the mutant-type protein sequence, and wherein the attention score is utilized in assessing importance of each amino acid of the wild-type protein sequence and the mutant-type protein sequence with respect to a protein stability.

11. The system as claimed in claim 7, wherein the pre-trained model is a protein language model (PLM) trained on one or more unlabeled protein sequences from a Protein database in a self-supervised manner using a mask language modeling principle which takes protein sequence as input and gives an appropriate contextual representation for each position in the protein sequence.

12. The system as claimed in claim 7, wherein the wild-type protein sequence, the mutant-type protein sequence, and the SMP in the wild-type protein sequence are processed by a neural network model comprising a protein language based feature extraction block and a neural network based prediction block predict protein stability by measuring the change in free energy (ΔΔG).

13. One or more non-transitory machine-readable information storage mediums comprising one or more instructions which when executed by one or more hardware processors cause:
receiving (a) a wild-type protein sequence, (b) a mutant-type protein sequence, and (c) a single mutation position (SMP) in the wild-type protein sequence;
extracting a plurality of features, by processing (a) the wild-type protein sequence, (b) the mutant-type protein sequence, and (c) the SMP in the wild-type protein sequence using a pre-trained protein language model;
separately generating
(i) a first representation matrix and (ii) a first attention matrix, from a plurality of extracted features of the wild-type protein sequence, and
(i) a second representation matrix and (ii) a second attention matrix, from the plurality of extracted features of the mutant-type protein sequence;
selecting using a selection algorithm one or more attention scores, by processing the first representation matrix, the first attention matrix, the second representation matrix, the second attention matrix, and the SMP, wherein the one or more attention scores are computed to obtain
a) a plurality of highly attended amino acids from the wild-type protein sequence with respect to a wild-type residue at the SMP, and
b) a plurality of highly attended amino acids from the mutant-type protein sequence with respect to a mutant-type residue at the SMP;
obtaining a residue environment by computing a first final feature representation, and a second final feature representation wherein,
the first final feature representation aggregates one or more vector representations of each of the plurality of highly attended amino acids and a SMP representation of the wild-type protein sequence extracted from the first representation matrix, and
the second final feature representation aggregates one or more vector representations of each of the plurality of highly attended amino acids and the SMP representation of the mutant-type protein sequence extracted from the second representation matrix;
computing a difference vector between the first final feature representation, and the second final feature representation, wherein the difference vector represents a change in amino acid residue and a change in residue environment after a single point mutation of the wild-type protein sequence; and
estimating a change in free energy (ΔΔG) by providing the difference vector to a neural-network based prediction block, wherein value of ΔΔG below 0 indicates that the single point mutation is stable with respect to the wild-type protein sequence.

14. The one or more non-transitory machine-readable information storage mediums as claimed in claim 13, wherein the first representation matrix is a feature vector representation of each amino acid in the wild-type protein sequence, and the second representation matrix is the feature vector representation of each amino acid in the mutant-type protein sequence.

15. The one or more non-transitory machine-readable information storage mediums as claimed in claim 13, wherein the first attention matrix captures attention paid by each amino acid residue to one or more other amino acids in the wild-type protein sequence, and the second attention matrix captures attention paid by each amino acid residue to one or more amino acids in the mutant-type protein sequence.
